# EUROPEAN PATENT SPECIFICATION

(11) **EP 1 864 672 B1**
(45) Date of publication and mention of the grant of the patent: **03.08.2016**
(21) Application number: 06728835.7
(22) Date of filing: 09.03.2006
(51) Int. Cl.: A61K 36/28, A61M 11/00, A61P 3/04

(54) **Appetite-suppressing agent and evaporator device thereofore**
Appetitzügler und Verdunstungsvorrichtung dafür
Agent coupe-faim et dispositif d'évaporation correspondant

(30) Priority: 29.03.2005 JP 2005095721
(43) Date of publication of application: 12.12.2007
(73) Proprietor: Yamashita, Hiromichi, Suita-shi, Osaka 565-0851 (JP)
(72) Inventor: Yamashita, Hiromichi, Suita-shi, Osaka 565-0851 (JP)
(74) Representative: Grünecker Patent- und Rechtsanwälte PartG mbB
(86) International application number: PCT/JP2006/304618
(87) International publication number: WO 2006/103895

(56) References cited:
- WO-A1-03/015765
- WO-A1-03/079830
- JP-A- 2002 179 581
- JP-A- 2003 274 919
- JP-A- 2004 256 522
- JP-A- 2004 359 606
- ADEBOYE J O ET AL: "Diuretic and antidiuretic activity of the leaf extracts of Vernonia cinerea (Less) (Fam. compositae)", PHYTOTHERAPY RESEARCH 199709 GB LNKD- DOI:10.1002/(SICI)1099-1573(199709)11:6<45 4::AID-PTR110>3.0.CO;2-1, vol. 11, no. 6, September 1997 (1997-09), pages 454-456, XP002630369, ISSN: 0951-418X
- LATHA R MARY ET AL: "Effect of Vernonia cinerea less flower extract in adjuvant-induced arthritis", GENERAL PHARMACOLOGY, vol. 31, no. 4, October 1998 (1998-10), pages 601-606, XP002630370, ISSN: 0306-3623
- DATABASE BIOSIS [Online] BIOSCIENCES INFORMATION SERVICE, PHILADELPHIA, PA, US; IGILE G O ET AL: "Nutritional assessment of Vernonia amygdalina leaves in growing mice", XP002155813, retrieved from BIOSIS PAN - PREV199598476864
- AKAH P A ET AL: "Blood sugar lowering effect of Vernonia amygdalina Del, in an experimental rabbit model", PHYTOTHERAPY RESEARCH 1992 GB, vol. 6, no. 3, 1992, pages 171-173, XP002630371, ISSN: 0951-418X
- AMOLE O O ET AL: "Toxicity studies of the aqueous extract of Vernonia amygdalina", BIOMEDICAL RESEARCH (ALIGARH), vol. 17, no. 1, January 2006 (2006-01), pages 39-40, XP008134867, ISSN: 0970-938X
- TANDON M. ET AL.: 'Insect antifeedant principles from Vernonia cinerea' PHYTOTHERAPY RESEARCH vol. 12, no. 3, 1998, pages 195 - 199, XP003000903

## Description

### TECHNICAL FIELD

This invention relates to anorectic agents suitable for diet, a diet method using the same, and an air regulator for diet containing an anorectic agent.

### BACKGROUND ART

Today, obesity poses a problem as a serious contemporary disease and various diet methods have been proposed and tried for health and beauty. Known diet methods include restriction of amounts and kinds of food, exercises and use of diet drugs.

Diet drugs can be classified by their function into anorectic agents, fat absorption restricting agents, sugar absorption restricting agents, fat burning agents, etc.

The inventor of the present application has proposed a diet drink containing leaves of Perilla frutescens crispa, naturally brewed vinegar and soil microorganisms (Patent document 1).

Also, tobacco filters or cigarettes which help to stop or reduce smoking are known which contain extracts of Vernonia cinerea, though they are not directly related to diet (Patent document 2).

Patent document 1: Japanese Patent 2931797
Patent document 2: Japanese patent publication 2003-274919A

ADEBOYE J.O. et al: "Diuretic and antidiuretic activity of the leaf extracts of Veronia cinerea (Less) (Fam. Compositae)", PHYTOTHERAPY RESEARCH 199709 GB LNKD-DOI:10.1002/(SICI) 1099-1573(199709)11:6 454::AID-PTR1103.0.CO;2-1, vol. 11, no. 6, September 1997 (1997-09), pages 454-456, ISSN: 0951-418X, discloses the preparation of an aqueous solvent extract of Veronia cinerea and its anti-diuretic activity.

JP2003274919A discloses aqueous solvent extracts of Veronia cinerea.

DATABASE BIOSIS [Online] BIOSCIENCES INFORMATION SERVICE, PHILADELPHIA, PA, US; IGILE G:O: et al: "Nutritional assessment of Veronia amygdalina leaves in growing mice", XP002155813, retrieved from BIOSIS PAN - PREV199598476864, reports that feeding by Veronia amygdalina leaves or an alcoholic extract of said leaves causes reduction of body weight gain in growing mice.

### DISCLOSURE OF THE INVENTION

### PROBLEMS TO WHICH THE INVENTION SEEKS A SOLUTION

But conventional diet methods such as exercises often require extraordinary efforts and patience and involve unpleasantness. For this reason, many people soon give up such a diet.

Also, conventional diet drugs, which serve to suppress appetite, are taken through digestive organs. Restricting the absorption of fat or sugar can cause health problems such as nutrition deficiency or other harmful side effects. Thus, such diet drugs do not always allow dieters to lose weight in a healthy manner. Also, continued malnutrition due to chronic indigestion may cause excessive dieting.

Other diet methods include a kind of aromatherapy in which people inhale what is called "appetite suppressing aroma". But different people prefer different aromas, so that selection of an aroma for each individual is difficult. Anyone in a room where someone else is inhaling a certain aroma has to inhale the same aroma irrespective of whether or not he or she likes it. This can cause trouble among family members or members of other groups.

An object of the present invention is to provide an anorectic agent, a diet method and an appetite suppressing apparatus with which one can suppress appetite properly without physical or mental burdens or excessive efforts and without any detrimental side effect and can lose weight safely and healthily.

Another object of the present invention is to provide an odorless anorectic agent which does not produce any aroma that may be offensive to certain people and which is harmless even if people who are not obese and thus does not need diet are forced to inhale it, and to provide novel diet method and air regulator for diet which use the same.

### MEANS TO ACHIEVE THE OBJECTS

According to the present invention, there is provided an anorectic agent which contains an aqueous solvent extract of Vernonia cinerea as an active ingredient. In particular, the present invention provides an anorectic agent for use in the treatment of obesity in accordance with claims 1 and 2, a non-medical diet method in accordance with claim 3, and air regulator for use in the treatment of obesity in accordance with claims 4 and 5, the use of an air regulator for non-medical dieting in accordance with claims 6 and 7, and an air regulator for diet in accordance with claim 8.

It is presumed that the active ingredient contained in the aqueous solvent extract of Vernonia cinerea is not absorbed but decomposed in digestive organs if taken orally. Only if taken directly into blood through respiratory organs such as lungs and nose mucous membrane, it is considered that it will act on the central nervous system through spinal liquid in an effective concentration.

As will be seen from the below-mentioned experiment results, the active ingredient of the aqueous solvent extract of Vernonia cinerea functions as an appetite suppressant on a person who is on diet to dieting.

As a result, a weight reducing effect will appear. But it will not have any effect on a person who does not want to lose weight. Therefore, it is safe even for children in the growth period.

Such an anorectic function and diet effect resulting therefrom will reveal more effectively by use of an anorectic agent containing aqueous solvent extracts of Vernonia cinerea and mulberry as active ingredients.

The use of mulberry with Vernonia cinerea suppresses the increase in the blood sugar level by the effect of water-soluble active ingredients contained in mulberry and provides a weight reducing effect more efficiently by the synergetic effect of the active ingredients contained in mulberry and Vernonia cinerea.

The mixing ratio (by dry weight) of Vernonia cinerea to mulberry in a dry state should be 100:1 to 100:70, preferably 100:10 to 100:50, more preferably 100:30 to 100:35 so that the anorectic effect and the blood sugar level reducing effect of the anorectic agent will develop in a good balance by the synergetic effect. That is, if the ratio is below the above range, the blood sugar level reducing effect could hardly be expected, and no synergetic effect in suppressing appetite could be obtained. Also, the addition of mulberry over the above range would not provide further reduction in the blood sugar level and thus meaningless.

The anorectic agent according to the present invention may be provided in a form in which the aqueous solvent extract is carried on a permeable solid carrier or a semi-solid carrier containing an aqueous solvent.

By carrying the aqueous solvent extract on such a solid or semi-solid carrier, the anorectic agent according to the present invention can be placed indoors in a simple container or mounted in a heating evaporator for forced evaporation or in an atomizer or a blower for easy use.

An anorectic effect and a weight reduction effect can be obtained reliably by dieting in which the anorectic agent according to the present invention is evaporated and the vapor produced is inhaled to absorb its active ingredient through respiratory organs.

As an apparatus used for diet for absorbing the active ingredient through respiratory organs, an air regulator for diet may be used which regulates air containing the anorectic agent by carrying the anorectic agent on a permeable solid carrier or a semi-solid carrier containing an aqueous solvent and passing an air flow through the carrier or heating the carrier to evaporate the active ingredient.

As another apparatus used for diet for absorbing the active ingredient through respiratory organs, an air regulator for diet may be used which regulates air containing the anorectic agent in the form of a liquid agent and evaporating the liquid agent by heating or by high-frequency oscillation.

By inhaling the air regulated by these apparatus, the active ingredient contained in the aqueous solvent extract of Vernonia cinerea will be taken directly into blood through respiratory organs such as lungs and nose mucous membrane. Therefore, an anorectic function and a weight reducing effect can be obtained reliably. If the anorectic agent contains a mulberry ingredient as a water-soluble active ingredient, the increase in the blood sugar level will be suppressed and a weight reducing effect will reveal more efficiently by the synergetic effect of the aqueous solvent extracts of mulberry and Vernonia cinerea.

### ADVANTAGES OF THE INVENTION

Because the anorectic agent according to the present invention contains an aqueous solvent extract of Vernonia cinerea as an active ingredient if the active ingredient is efficiently taken into blood, the user can control appetite and lose weight safely without metal or physical burdens, and achieve a weight reducing effect safely and healthily without requiring excessive efforts.

Because the active ingredient of the anorectic agent according to the present invention is inherently odorless, one can use it without worrying about other people in the same room. Thus, the anorectic agent according to the invention is versatile.

Because with the diet method according to the present invention, the active ingredient is taken into blood directly through respiratory organs by evaporating the anorectic agent in a liquid form and inhaling the vapor produced, the user hardly feels mental or physical burdens and the active ingredient acts on a central nervous system efficiently. Thus, the user's appetite is controlled properly, so that a weight reducing effect can be obtained safely and healthily. If an odorless one is used as the anorectic agent, the anorectic agent will never offend people around the user.

Also, the air regulator for diet according to the present invention evaporates the anorectic agent soaked in a porous material or evaporates the anorectic agent in a liquid state, and regulates the air containing the effective component, so that it can be taken through the respiratory organs of the user. By simply inhaling the thus produced air, the dieter can easily lose weight.

### BRIEF DESCRIPTION OF THE DRAWINGS

Fig. 1 is a schematic view of the air regulator for diet according to the present invention;
Fig. 2 is a graph showing the relationship between the average weights of mice in groups A to C and the testing time;
Fig. 3 is a graph showing the relationship between the weights of feed consumed by mice in groups A to C and the testing time; and
Fig. 4 is a graph showing the relationship between the average BMI values of human subjects in groups A to C and the testing time.

### DESCRIPTION OF NUMERALS

- 1: liquid agent
- 2: container
- 3: heater
- 4: control panel
- 5: thermostat
- 6: timer

### BEST MODE FOR EMBODYING THE INVENTION

The aqueous solvent extract of Vernonia cinerea used in the present invention can be obtained as described below.

Vernonia cinerea is an herb in the Asteraceae family, Vernonia genus. Its scientific name is Vernonia cinerea (Linn.) Less. It is produced as a material for herb tea. Thailand is a main producing country.

Vernonia cinerea is grown not only in Thailand but in countries in the subtropical zone that are similar in climate and growing environment to Thailand (such as Cambodia, Myanmar, Laos, Vietnam, Indonesia, China and India). The growing conditions are not particularly limited. Vernonia cinerea grown in an ordinary growing method may be used in the present invention. Its F1 hybrids, gene-manipulated varieties, other varieties or cultivated ones may also be used in the present invention.

Vernonia cinerea is preferably used in its entirety as a material for extraction, but may be used only partially. For example, its leaves, stems, flowers, etc. may be used singly or in combination. Its leaves are particularly suited to obtain dry powder of a desired particle diameter. In use, the material is preferably cut to a proper size for easy extraction and dried as necessary.

Mulberry is Morus L., which is a dicotyledon belonging to the Moraceae family. Ordinary such mulberry contains deoxynojirimycin, calcium, ß-carotene (vitamin A), γ-amino acid, flavonoid, potassium, iron, other amino acids, etc.

For example, mulberry may be Morus alba L.; Morus atropurpure Roxb., Morus bombycis Koidz., Morus alba L. var stylosa Bur.; Morus japonica Bailey nonSieb., Morus australis Poir.; Morus acidosa Griff. Among these mulberries, the first two are easily available as cultivated varieties. As the extraction material, any portion of mulberry except its roots such as its leaves and stems can be used. But it is preferable to use its leaves only or its entirety except its roots.

In the present invention, an aqueous solvent may be used as an extraction solvent. As an aqueous solvent, water only or a mixture of water and a hydrophilic solvent may be used. The hydrophilic solvent may be alcohol, ketone or organic acid. The alcohol may be a lower alcohol such as ethanol, propanol, isopropanol or butanol. The ketone may be acetone. The organic acid may be acetic acid, propionic acid, butyric acid, citric acid, succinic acid, malic acid or lactic acid. Also, the extraction solvent may contain an organic solvent provided such an organic solvent does not impair the effect of this invention.

Preferably, the extraction solvent contains 1 to 15 grams, more preferably not less than 3 grams (further preferably not less than 6 grams) and not more than 12 grams of Vernonia cinerea based on 100 ml of the extraction solvent.

As for the extraction time, extraction is preferably continued after Vernonia cinerea has been put into 100 ml of water and boiled until the total amount decreases to about half (about 30 to 40 minutes), though depending on the solvent used and the amount of Vernonia cinerea. By doing so, an extract of a sufficient concentration to achieve a desired effect can be obtained.

For extraction, Vernonia cinerea may be put into the extraction solvent after the extraction solvent has been boiled, or Vernonia cinerea may be put into the extraction solvent and the mixture may be boiled. But it has been confirmed by experiment that the former method provides better results.

A desired extract is obtained by extracting Vernonia cinerea in this way, filtering it as necessary and leaving it to room temperature. Also, cold water extraction and high-pressure extraction may be adopted.

As for the extraction method, one of the following methods can be adopted: impregnating at room temperature or while cooling or heating and then extracting; extracting by distillation such as water vapor distillation; pressing method in which an extract is obtained by pressing; and carrying out one of these methods under reduced pressure. These methods may be used singly or in combination.

The thus obtained aqueous solvent extract may be used in a liquid state. Also, the liquid anorectic agent may be used carried on a permeable solid carrier or a semi-solid carrier containing an aqueous solvent.

The permeable solid carrier may be a sponge-like carrier of three dimensional reticular structure having pores communicating with each other, a foamed resin, activated charcoal, or a sintered body. Also, it may be a fibrous material such as a fiber bundle, braided fabric or non-woven fabric. The semi-solid carrier may be jelly, agar or a super absorbent resin. The semi-solid carrier may be one which can carry a liquid in an evaporatable state.

The solid carrier may be impregnated into e.g. moxa or incense sticks, and dried and ignited to gradually evaporate the active components.

Vernonia cinerea and mulberry are vegetables known for applications in which their extracts are taken orally in the form of tea leaves or herbs. The extracts of their aqueous solvents are therefore not poisonous at all even if taken orally. It is also apparent from experience that their vapors are safe to smell or inhale.

The aqueous solvent extract of Vernonia cinerea used for the anorectic agent according to the present invention contains not only particular known ingredients of Vernonia cinerea but other unspecific ingredients thereof. It is therefore difficult to determine the amounts of its active ingredients. But based on the clinical experiments using mice as described below, it is considered that if the extract (i.e. aqueous solvent extract) of Vernonia cinerea is obtained by putting 30 g of chips of Vernonia cinerea in 1000 ml of water, heating and boiling the mixture until the amount of water reduces to about half the original weight, and filtering the mixture, an adult weighing 60 kg should take such an extract by respiration in an amount of not less than 0.05 ml, preferably 0.1 to 3.0 ml, that is, not less than 0.001 ml, preferably 0.002 to 0.05 ml per kilogram of body weight for best results.

This amount is much smaller than the amount of such an extract that has to be taken orally to obtain best results. This is one of the most important advantages of the present invention. The reason why it is possible to drastically reduce the amount of the extract taken if taken by respiration is because by taking the extract by respiration, its active ingredients are directly absorbed into the body while bypassing the steps of decomposition by enzymes, absorption and resynthesis, which are necessary if the extract is taken orally.

An apparatus embodying the invention which is used to absorb the active ingredients of the extract according to the invention through the respiratory organs is now described with reference to the accompanying drawings.

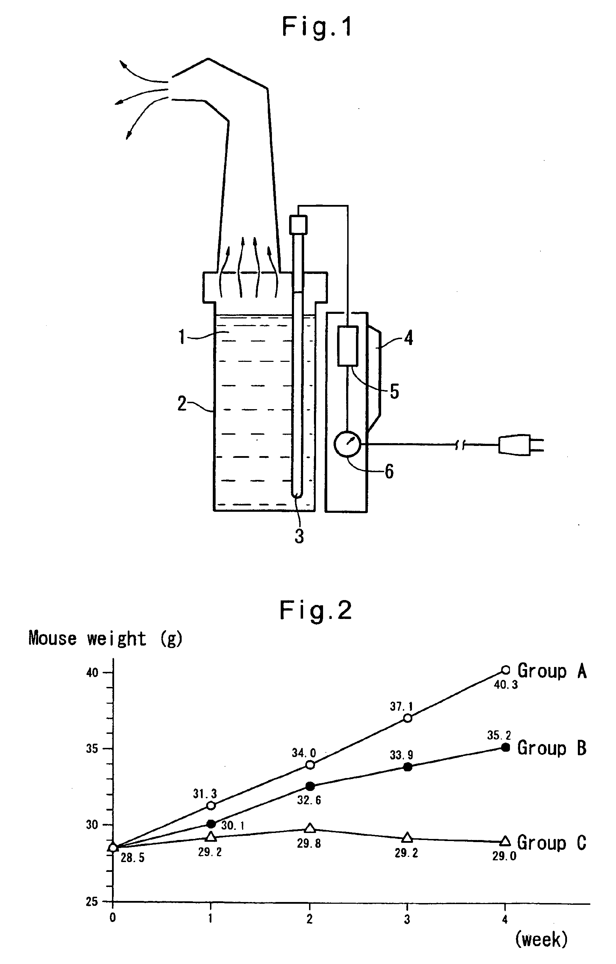

As shown in Fig. 1, an air regulator for diet embodying the present invention includes a container 2 containing a liquid-state anorectic agent 1 according to the invention, a heater 3 for heating the liquid agent 1 to evaporate it, thereby regulating concentration of the liquid agent in the room air.

The heater 3 may be a known type such as an electric heater controlled by a control panel 4. The heater 3 is connected to an AC outlet through a thermostat 5 and a timer 6 for selectively turning on and off the heater to control the air temperature and the atomizing time and to prevent overheating.

The air regulator evaporates a controlled amount of the liquid agent 1 for a desired time period to fill the room with the vapor of the liquid agent 1. Anyone in the room can inhale the vapor of the liquid agent 1, i.e. the aqueous solvent extract of Vernonia cinerea according to the invention.

Instead of heating the entire liquid agent in the container as described above, part of the liquid agent may be evaporated by high-frequency oscillation like a known humidifier. Also, the liquid agent 1 may be evaporated by impregnating a fibrous or porous carrier with the liquid agent and passing air from a fan through the carrier so that one can inhale the air containing the vapor produced.

The carrier may be a fiber bundle such as a cigarette filter. Such a fiber bundle may be put in a small container having a mouth end such as a cigarette-shaped perfume sucking tool. By sucking such a fiber bundle, one can take the liquid agent 1. Also, such a fiber bundle or any other permeable solid carrier may be a plate. By heating the plate, the active ingredients of the liquid agent are evaporated. Further, the aqueous solvent extract may be adhered to a carrier in the form of a non-woven fabric or to a carrier of a filter type, and the carrier may be mounted on an air outlet of an air conditioner or an electric fan so that the air flow will pass through the carrier.

Also, it is possible to efficiently evaporate the liquid agent by dipping part of a rod-shaped carrier formed of carbon fiber or a sintered body in the liquid agent in a container to wet the entire carrier by capillary action, heating the exposed portion of the carrier with a heater to accelerate evaporation, and bringing the air flow from a blower into contact with the exposed portion of the carrier as necessary.

Both the aqueous solvent extract of Vernonia cinerea and the extract of mulberry are odorless when their vapors are inhaled. They may be used as it is without adding any flavoring. In this regard, the diet method according to the present invention differs from aromatherapy, which causes some action by psychological effects through aroma. It is considered that in the diet method according to the invention, the odorless active ingredients of the extract migrate from the bloodstream into the spinal cord liquid and acts on the brain directly. Thus, this diet method does not have a weight reducing effect by psychological effects through aroma like aromatherapy.

Typical materials used to treat obesity in aromatherapy include fennel, juniper and patchouli. Vernonia cinerea is not generally used in aromatherapy for treating obesity. Also, there is no literature, theory or report showing that Vernonia cinerea has an anti-corpulence effect when taken as a drug, a drink or a food.

As described above, the aqueous solvent extract of Vernonia cinerea and the extract of mulberry are intrinsically odorless and do not have any compulsory psychological effects through aroma. In this regard, the diet method according to the invention differs from aromatherapy.

Unpleasant odors such as odors of feces and urine can cause anyone to lose appetite and thus to lose weight. But no one will want to be exposed to such unpleasant odors. Odors used for aromatherapy are not necessarily pleasant to anyone, and may be offensive to some people. In contrast, because the aqueous solvent extract of Vernonia cinerea according to this invention is odorless, it does not give any unpleasant feeling about odor. Therefore, it can be used by anyone without giving unpleasant feeling and is versatile.

### [EXAMPLES]

An aqueous solvent extract of Vernonia cinerea used for experiments in the Examples was prepared in the following manner.

First, 30 g of chips of Vernonia cinerea were mixed into 1000 ml of water and the mixture was heated and boiled until the amount of water reduces to about half the original weight. By filtering the mixture, an aqueous solvent extract of Vernonia cinerea was obtained.

In order to test the aqueous solvent extract of Vernonia cinerea for anti-corpulent function to mice, ordinary grown-up mice were fed with high-fat feed for four weeks to check the anti-corpulence function of the anorectic agent of the Example.

### <Anti-corpulence function test for the anorectic agent>

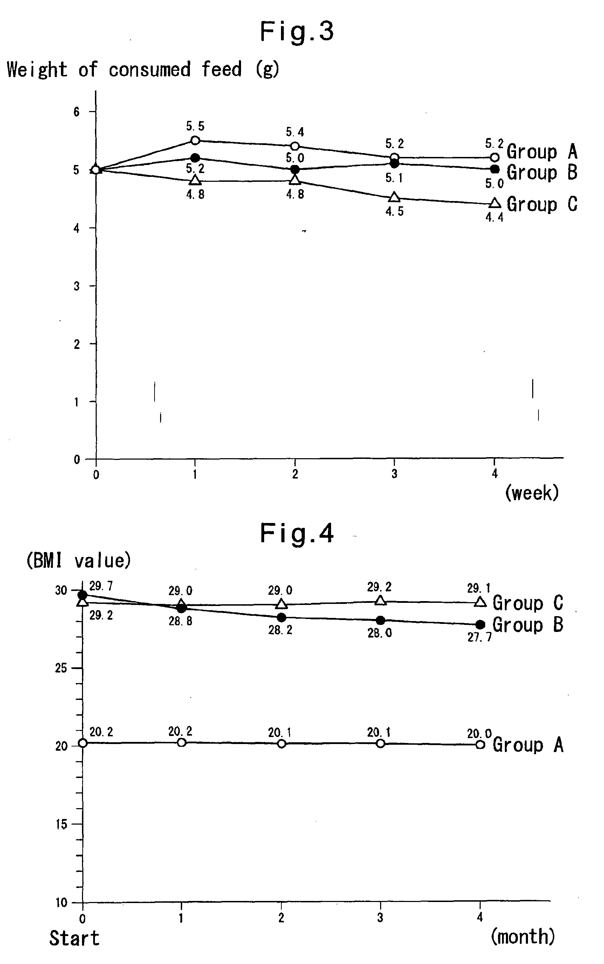

1. Animals used: 60 ordinary mice (female, 12 weeks old) were tamed for one week, and after confirmation that there was no increase in weight of any of the mice, subjected to the test. Immediately before the test, the mice were divided into three groups A, B and C, 20 in each group, so that the average weights of the respective groups are equal to each other.
2. Breeding environment: Confined in a reinforced concrete building; temperature: 23±3 degrees C; humidity: 40 to 60%
3. Breeding conditions: Every five mice were put in one cage with wood chips (KAC) placed on the floor, which were changed once in a week. Water was given freely from a water supply bottle. Lights were alternately turned on and off at 12-hour intervals from the start of taming to the end of the experiment.
4. Specimens: The liquid used for groups B and C was an anorectic agent containing the aqueous solvent extract according to the present invention. The mice of group B took it by drinking and those of group C took it by inhaling.
5. Feed: During the taming period (one week before the experiment) ordinary powder feed (made by CLEA Japan, Inc. CE-2) was used. But upon the start of the experiment, high-fat feed for research of corpulence and diabetes (Quick Fat made by CLEA Japan, Inc.) was used for all groups. For any cage, an average of 5 g was given per mouse. Feeders were checked every 6 hours and a suitable amount of feed was put in any feeder that was empty.
6. Testing method: The mice of group A (control group) was fed with high-fat feed only. The mice of group B was given drinking water containing 0.5 % of the anorectic agent of the Example. In this case, an intake per day is 0.005 mg for a mouse weighing 30 g. This is equivalent to drinking 30 ml of the anorectic agent of the Example for an adult weighing 60 kg and drinking 2000 ml of water per day. Before the experiment, it was confirmed that the mice drank the mixture liquid freely.
   The mice of group C were fed with high-fat feed like the mice of group A. By use of a small heating evaporator installed at a distance of about 30 cm from every cage, about 0.5 ml per day of the anorectic agent of the Example was evaporated for natural inhalation by the mice of group C, and its effect was determined.
   The effect was determined by measuring the mice weights and the weight of the feed consumed once a week. Figs. 2 and 3 show the results.
   The dosage of 0.5 ml per day given to the group C mice by evaporation is considered to be rather high for the weight of mice. But because most of the evaporated agent is dispersed into the air without being inhaled by the mice, the effect of the anorectic agent will scarcely appear if the dosage by evaporation is reduced to a lower level. Also, because this experiment is not for determining the effect of the anorectic agent on the prevention of corpulence but for determining its effect on the weight reduction, not corpulent diabetic mice (kk-AyTa mouse) but ordinary mice were used. In order to avoid cannibalism, all of the mice used were 12-week-old, grown-up female mice.
7. Determination of Effect: Figs. 2 and 3 clearly show that the increase in weight of the mice of Group C was remarkably low, without the need for statistical processing. Figs. 2 and 3 show weight reductions in weeks 3 and 4. Although their average weight did not fall below the level at the start of the experiment, it approached this level.

This is a desired result in that the average weight decreased to the initial ideal level but has not decreased excessively below the ideal level. Only the mice of Group C showed reductions in the feed consumption in weeks 3 and 4, during which their average weight began to decrease.

On the other hand, the mice of Group B showed a gradual increase in weight, though not so excessive as the mice of Group A. Their feed consumption did not decrease, either.

These results show that the anorectic agent containing the aqueous solvent extract of Vernonia cinerea according to the invention as its active ingredient has little diet effect when taken orally as a drink, and has a remarkable diet effect only when inhaled in the form of a vapor.

Next, to determine the diet effect of the anorectic agent according to the invention on human subjects, monitor groups each consisting of 20 people were subjected to the following experiment.

The subjects were divided into the monitor groups according to their BMI (Body Mass Index) values, which are [body weight (kg)/ height (m)]². Subjects having a BMI value of 25 or over were determined to be obese, and those having a BMI value of less than 18 were determined to be thin.
Group A: For nonobese subjects, i.e. those having a BMI value of less than 25, the air regulator for diet shown in Fig. 1 was installed near the head of each subject every night while they were sleeping to evaporate 3 ml of the extract of Vernonia cinerea of the Example so that the subjects inhale the extract while sleeping. The results of the experiment are shown in Table 1.
Group B: For obese subjects, i.e. those having a BMI value of 25 or over, the air regulator for diet shown in Fig. 1 was installed near the head of each subject every night while they were sleeping to evaporate 3 ml of the extract of Vernonia cinerea of the Example so that the subjects inhale the extract while sleeping. The results of the experiment are shown in Table 2.
Group C: Obese subjects, i.e. those having a BMI value of 25 or over drank 30 ml of the extract of Vernonia cinerea of the Example before going to sleep and after waking up. The results of the experiment are shown in Table 3.

**Table 1**

| Group A | Sex | Age | Weight | Height | BMI | Month 1 | Month 2 | Month 3 | Month 4 |
|---|---|---|---|---|---|---|---|---|---|
| 1 | Female | 73 | 41.5 | 1.52 | 18.0 | 17.7 | 17.7 | 17.7 | 17.7 |
| 2 | Male | 67 | 53.0 | 1.685 | 18.7 | 18.5 | 18.5 | 18.5 | 18.1 |
| 3 | Male | 45 | 68.0 | 1.71 | 23.3 | 23.1 | 23.1 | 23.1 | 22.9 |
| 4 | Male | 40 | 65.0 | 1.73 | 22.2 | 22.2 | 22.2 | 21.6 | 21.6 |
| 5 | Male | 45 | 66.0 | 1.75 | 21.6 | 21.6 | 21.4 | 21.4 | 21.4 |
| 6 | Female | 36 | 43.0 | 1.63 | 16.2 | 16.4 | 16.6 | 16.6 | 16.4 |
| 7 | Male | 45 | 65.0 | 1.71 | 22.2 | 22.2 | 22.1 | 22.1 | 22.1 |
| 8 | Female | 48 | 43.0 | 1.54 | 18.1 | 18.1 | 17.9 | 17.9 | 17.9 |
| 9 | Female | 60 | 41.0 | 1.49 | 18.4 | 18.4 | 18.4 | 18.2 | 18.4 |
| 10 | Male | 54 | 69.0 | 1.76 | 22.3 | 22.1 | 22.1 | 22.1 | 22.3 |
| 11 | Female | 44 | 61.0 | 1.66 | 22.1 | 22.0 | 22.1 | 22.0 | 22.0 |
| 12 | Male | 37 | 68.0 | 1.72 | 23.0 | 24.3 | 22.8 | 23.0 | 22.8 |
| 13 | Female | 58 | 43.0 | 1.58 | 17.2 | 17.6 | 17.8 | 17.6 | 17.8 |
| 14 | Male | 56 | 65.0 | 1.74 | 21.5 | 21.5 | 21.3 | 21.5 | 21.3 |
| 15 | Male | 43 | 67.5 | 1.72 | 23.0 | 23.0 | 22.6 | 23.0 | 22.6 |
| 16 | Female | 40 | 47.0 | 1.56 | 19.0 | 19.3 | 19.1 | 19.1 | 19.1 |
| 17 | Male | 45 | 59.0 | 1.73 | 19.7 | 19.7 | 19.7 | 19.7 | 19.7 |
| 18 | Male | 44 | 46.0 | 1.60 | 18.0 | 18.1 | 18.1 | 18.1 | 18.1 |
| 19 | Female | 70 | 46.5 | 1.57 | 19.1 | 19.1 | 19.2 | 19.2 | 19.1 |
| 20 | Male | 16 | 49.0 | 1.56 | 20.2 | 20.2 | 20.2 | 20.2 | 20.2 |
| | | | | Average | 20.2 | 20.2 | 20.1 | 20.1 | 20.0 |

**Table 2**

| Group B | Sex | Age | Weight | Height | BMI | Month 1 | Month 2 | Month 3 | Month 4 |
|---|---|---|---|---|---|---|---|---|---|
| 1 | Male | 62 | 75.0 | 1.68 | 26.6 | 25.9 | 25.1 | 24.8 | 24.8 |
| 2 | Female | 62 | 68.0 | 1.56 | 27.9 | 26.7 | 26.2 | 25.8 | 25.4 |
| 3 | Female | 57 | 67.0 | 1.55 | 27.9 | 26.6 | 26.2 | 25.4 | 25.4 |
| 4 | Male | 46 | 82.0 | 1.71 | 28.0 | 27.0 | 26.3 | 26.3 | 26.3 |
| 5 | Female | 39 | 66.0 | 1.58 | 26.4 | 25.2 | 24.4 | 24.8 | 24.4 |
| 6 | Male | 62 | 81.0 | 1.70 | 28.0 | 27.0 | 26.6 | 26.3 | 26.0 |
| 7 | Female | 71 | 89.0 | 1.57 | 36.1 | 34.9 | 34.0 | 33.7 | 33.3 |
| 8 | Female | 39 | 70.0 | 1.65 | 25.7 | 24.6 | 25.0 | 25.0 | 24.6 |
| 9 | Female | 60 | 73.5 | 1.58 | 29.2 | 28.0 | 27.2 | 27.2 | 26.4 |
| 10 | Male | 49 | 75.0 | 1.59 | 29.7 | 28.5 | 28.0 | 27.6 | 28.0 |
| 11 | Female | 75 | 69.0 | 1.54 | 29.1 | 27.8 | 27.0 | 27.0 | 26.6 |
| 12 | Female | 61 | 72.5 | 1.58 | 28.8 | 28.0 | 27.2 | 27.2 | 26.8 |
| 13 | Male | 42 | 91.0 | 1.71 | 31.1 | 30.1 | 29.4 | 28.7 | 28.4 |
| 14 | Female | 73 | 89.0 | 1.54 | 37.5 | 36.3 | 35.4 | 34.2 | 34.2 |
| 15 | Female | 52 | 66.0 | 1.53 | 28.2 | 26.9 | 26.5 | 26.5 | 26.9 |
| 16 | Female | 74 | 87.5 | 1.59 | 34.4 | 33.6 | 33.4 | 32.8 | 32.0 |
| 17 | Female | 70 | 78.5 | 1.56 | 32.3 | 32.3 | 31.6 | 31.6 | 31.6 |
| 18 | Female | 78 | 66.0 | 1.48 | 30.1 | 29.7 | 29.2 | 28.7 | 28.3 |
| 19 | Female | 56 | 66.5 | 1.57 | 26.8 | 26.8 | 26.4 | 26.8 | 26.4 |
| 20 | Female | 36 | 78.0 | 1.60 | 30.5 | 29.3 | 28.9 | 28.9 | 28.5 |
| | | | | Average | 29.7 | 28.8 | 28.2 | 28.0 | 27.7 |

**Table 3**

| Group C | Sex | Age | Weight | Height | BMI | Month 1 | Month 2 | Month 3 | Month 4 |
|---|---|---|---|---|---|---|---|---|---|
| 1 | Female | 63 | 69.5 | 1.54 | 29.3 | 29.1 | 29.1 | 29.3 | 29.3 |
| 2 | Female | 59 | 68.0 | 1.52 | 29.4 | 29.2 | 29.2 | 29.0 | 29.0 |
| 3 | Male | 51 | 90.5 | 1.72 | 30.5 | 30.4 | 30.3 | 30.3 | 29.7 |
| 4 | Female | 40 | 92.0 | 1.59 | 36.4 | 36.2 | 36.2 | 36.4 | 36.4 |
| 5 | Male | 46 | 74.0 | 1.71 | 25.3 | 25.6 | 25.8 | 25.8 | 25.6 |
| 6 | Male | 56 | 81.0 | 1.72 | 27.4 | 27.0 | 27.4 | 27.4 | 27.4 |
| 7 | Female | 60 | 72.0 | 1.58 | 28.8 | 28.8 | 28.6 | 28.6 | 28.8 |
| 8 | Female | 24 | 62.0 | 1.55 | 25.8 | 25.8 | 25.8 | 25.8 | 25.8 |
| 9 | Female | 54 | 63.5 | 1.54 | 26.8 | 26.6 | 26.6 | 26.8 | 26.6 |
| 10 | Female | 49 | 92.0 | 1.65 | 33.8 | 33.8 | 34.5 | 33.8 | 33.4 |
| 11 | Female | 44 | 62.0 | 1.55 | 25.8 | 24.6 | 24.6 | 25.8 | 24.6 |
| 12 | Female | 55 | 76.0 | 1.58 | 30.4 | 30.2 | 30.2 | 30.0 | 30.0 |
| 13 | Female | 34 | 78.5 | 1.57 | 31.8 | 31.8 | 31.8 | 31.6 | 31.6 |
| 14 | Female | 67 | 63.0 | 1.54 | 26.6 | 27.0 | 26.6 | 26.3 | 26.3 |
| 15 | Female | 40 | 63.5 | 1.55 | 26.4 | 26.4 | 26.4 | 26.4 | 26.2 |
| 16 | Male | 61 | 77.0 | 1.71 | 26.3 | 26.2 | 26.2 | 26.3 | 26.2 |
| 17 | Female | 63 | 74.0 | 1.58 | 29.6 | 29.6 | 29.6 | 29.4 | 29.4 |
| 18 | Female | 36 | 75.5 | 1.59 | 29.9 | 29.7 | 29.7 | 29.9 | 29.7 |
| 19 | Male | 34 | 78.0 | 1.61 | 30.1 | 29.9 | 29.7 | 29.7 | 29.7 |
| 20 | Female | 59 | 78.5 | 1.54 | 33.1 | 32.9 | 32.9 | 32.7 | 32.2 |
| | | | | Average | 29.2 | 29.0 | 29.0 | 29.2 | 29.1 |

Tables 1 to 3 and Fig. 4 show that for Group A, there is little change in BMI value. This means that for subjects having standard or less-than-standard BMI values, there is little diet effect.

For Group C, in spite of the fact that the anorectic agent of the Example was used in an amount greater by about 20 times than for Group B, little change in BMI was observed. This shows that there is no diet effect if taken orally.

On the other hand, for Group B only, the BMI value decreased significantly. That is, the average BMI value decreased from 29.7 to 27.7 in four months. This shows that the inhalation of the anorectic agent of the Example is effective. Also, reviewing the individuals in Group B in detail, it is seen that the use of the Example for subjects of standard or less-than-standard weights resulted in little decrease in weight, and the use for obese subjects only was effective. This shows that the Example is an anorectic agent which provides a weight reducing effect safely and healthily without any side effect by controlling appetite safely by oneself.

Considering these results, if a conventional anorectic agent is evaporated into the air so that a person who wants to lose weight can inhale its vapor, other people in the same room will also inhale the vapor, not knowing that they are inhaling the vapor. This is dangerous if such other people are too lean, suffer from anorexia, or children in the growth period.

But the anorectic agent according to the present invention will have no such detrimental effects on people who must not lose weight or children in the growth period, who have to eat sufficiently. Also, it is odorless and can be inhaled in a pleasant atmosphere. It is ideally convenient, safe and pleasant and permits diet without burden.

The extract of Vernonia cinerea was subjected to reversed phase chromatography and its analysis curve was obtained. The ingredients appearing at peaks 11.984 seconds and 13.741 seconds, which were presumably particularly active ingredients, were used as active ingredients of the anorectic agent. As a result, they induced loss of appetite for all the subjects. But we did not collect data when these particular active ingredients are used for a long period of time as in Tables 1, 2 and 3. This is because we refrained from using such particular active ingredients for humans continuously for a month or longer in consideration of safety.

From these results, it is considered that the anorectic agent according to the present invention, which contain all the ingredients of the aqueous solvent extract of Vernonia cinerea is a safe and excellent anorectic agent.

## Claims

1. An anorectic agent for use in the treatment of obesity containing an aqueous solvent extract of Vernonia cinerea as an active ingredient, wherein the anorectic agent is administered by inhalation.

2. The anorectic agent for use in the treatment of obesity according to claim 1, wherein a liquid component of the anorectic agent of claim 1 in liquid form is evaporated and the vapor produced is inhaled by an obese person who wants to diet.

3. A non-medical diet method wherein a liquid component of the anorectic agent of claim 1 in liquid form is evaporated and the vapor produced is inhaled by a person who wants to diet.

4. An air regulator for use in the treatment of obesity comprising a means for carrying said aqueous solvent extract of claim 1 on a breathable solid carrier or a semi-solid carrier containing an aqueous solvent and passing an air flow through said carrier or heat said carrier to evaporate said active ingredient, thereby regulating air containing the anorectic agent.

5. An air regulator for use in the treatment of obesity which evaporates a liquid containing the anorectic agent of claim 1 by heating or high-frequency oscillation, thereby regulating air containing the anorectic agent.

6. Use of an air regulator for non-medical dieting, wherein the air regulator comprises a means for carrying said aqueous solvent extract of claim 1 on a breathable solid carrier or a semi-solid carrier containing an aqueous solvent and passing an air flow through said carrier or heat said carrier to evaporate said active ingredient, thereby regulating air containing the anorectic agent.

7. Use of an air regulator for non-medical dieting, wherein the air regulator evaporates a liquid containing the anorectic agent of claim 1 by heating or high-frequency oscillation, thereby regulating air containing the anorectic agent.

8. An air regulator for diet which evaporates a liquid containing the anorectic agent of claim 1 by high-frequency oscillation, thereby regulating air containing the anorectic agent.

## Patentansprüche

1. Appetitzügler zur Verwendung in der Behandlung von Fettleibigkeit, enthaltend einen wässrigen Lösungsmittelextrakt von *Veronica cinerea* als ein aktiver Bestandteil, wobei der Appetitzügler durch Inhalation verabreicht wird.

2. Der Appetitzügler zur Verwendung in der Behandlung von Fettleibigkeit gemäß Anspruch 1, wobei eine flüssige Komponente des Appetitzüglers von Anspruch 1 in flüssiger Form verdampft wird und der hergestellte Dampf von einer fettleibigen Person, die Diät machen will, inhaliert wird.

3. Nichtmedizinisches Diätverfahren, wobei eine flüssige Komponente des Appetitzüglers von Anspruch 1 in flüssiger Form verdampft wird, und der hergestellte Dampf von einer Person, die Diät machen will, inhaliert wird.

4. Luftregulator zur Verwendung in der Behandlung von Fettleibigkeit, umfassend ein Mittel zum Trägern von besagtem wässrigen Lösungsmittelextrakt von Anspruch 1 auf einen atmungsaktiven festen Träger oder einen semi-festen Träger, enthaltend ein wässriges Lösungsmittel, und Durchströmen eines Luftflusses durch besagten Träger oder Erhitzen von besagtem Träger, um den besagten aktiven Bestandteil zu verdampfen, wodurch Luft, enthaltend den Appetitzügler, reguliert wird.

5. Luftregulator zur Verwendung in der Behandlung von Fettleibigkeit, der eine Flüssigkeit, enthaltend den Appetitzügler von Anspruch 1, durch Erhitzen oder Hochfrequenzoszillation verdampft, wodurch Luft, enthaltend den Appetitzügler, reguliert wird.

6. Verwendung eines Luftregulators zum Durchführen einer nichtmedizinischen Diät, wobei der Luftregulator ein Mittel zum Trägern von besagtem wässrigen Lösungsmittelextrakt von Anspruch 1 auf einem atmungsaktiven festen Träger oder einem semi-festen Träger, enthaltend ein wässriges Lösungsmittel, umfasst, und Durchströmen eines Luftflusses durch besagten Träger oder Erhitzen von besagtem Träger, um den besagten aktiven Bestandteil zu verdampfen, wodurch Luft, enthaltend den Appetitzügler, reguliert wird.

7. Verwendung eines Luftregulators zum Durchführen einer nichtmedizinischen Diät, wobei der Luftregulator eine Flüssigkeit, enthaltend den Appetitzügler von Anspruch 1, durch Erhitzen oder Hochfrequenzoszillation verdampft, wodurch Luft, enthaltend den Appetitzügler, reguliert wird.

8. Luftregulator zum Dürchführery einer Diät, der eine Flüssigkeit, enthaltend den Appetitzügler von Anspruch 1 durch Hochfrequenzoszillation verdampft, wodurch Luft, enthaltend den Appetitzügler, reguliert wird.

## Revendications

1. Agent anorexigène destiné à une utilisation dans le traitement de l'obésité, contenant un extrait de solvant aqueux de Vernonia cinerea comme ingrédient actif, l'agent anorexigène étant administré par inhalation.

2. Agent anorexigène destiné à une utilisation dans le traitement de l'obésité selon la revendication 1, dans lequel une composante liquide de l'agent anorexigène conforme à la revendication 1, sous forme liquide, est évaporée, et la vapeur produite est inhalée par une personne obèse qui souhaite faire un régime.

3. Procédé non médical de régime dans lequel une composante liquide de l'agent anorexigène conforme à la revendication 1, sous forme liquide, est évaporée, et la vapeur produite est inhalée par une personne qui souhaite faire un régime.

4. Régulateur d'air destiné à une utilisation dans le traitement de l'obésité comprenant un moyen permettant de transporter ledit extrait de solvant aqueux conforme à la revendication 1 sur un porteur solide ou sur un porteur a demi solide respirable contenant un solvant aqueux et faisant passer une circulation d'air au travers dudit porteur ou bien chauffant ledit porteur afin de faire évaporer ledit ingrédient actif, ce qui régule ainsi l'air contenant l'agent anorexigène.

5. Régulateur d'air destiné à une utilisation dans le traitement de l'obésité qui fait évaporer un liquide contenant l'agent anorexigène conforme à la revendication 1 par chauffage ou par oscillation à haute fréquence, ce qui régule ainsi l'air contenant l'agen.t anorexigène.

6. Utilisation d'un régulateur d'air pour un régime non médical, le régulateur d'air comprenant un moyen permettant de transporter ledit extrait de solvant aqueux conforme à la revendication 1 sur un porteur solide ou sur un porteur à demi solide respirable contenant un solvant aqueux et faisant passer une circulation d'air au travers dudit porteur ou chauffant ledit porteur afin d'évaporer ledit ingrédient actif, ce qui régule ainsi l'air contenant l'agent anorexigène.

7. Utilisation d'un régulateur d'air pour un régime non médical, le régulateur d'air faisant évaporer un liquide contenant l'agent anorexigène conforme à la revendication 1 par chauffage ou par oscillation à haute fréquence, ce qui régule ainsi l'air contenant l'agent anorexigène.

8. Régulateur d'air destiné à un régime qui fait évaporer un liquide contenant l'agent anorexigène conforme à la revendication 1 par oscillation à haute fréquence, ce qui régule ainsi l'air contenant l'agent anorexigène.
